# EUROPEAN PATENT APPLICATION

(11) **EP 2 133 364 A1**
(43) Date of publication of application: **16.12.2009**
(21) Application number: 08158074.8
(22) Date of filing: 11.06.2008
(51) Int. Cl.: C07K 14/78

(54) **Block Co-Polypeptide and hydrogels made thereof**

(71) Applicant: Stichting Dienst Landbouwkundig Onderzoek, 6701 BH Wageningen (NL)
(72) Inventor: The designation of the inventor has not yet been filed
(74) Representative: Hatzmann, Martin

(57) **Abstract**

Disclosed is a block co-polypeptide comprising at least two blocks T and at least one block S, with blocks T and S alternating, wherein each T independently denotes a Trimerizing Block being a polypeptide block capable of forming a thermoreversible collagen-like triple helix structure, and wherein each S independently denotes a Spacer Block S being a polypeptide neither forming a collagen-like triple helix. The block co-polypeptide is capable of forming a gel, and is useful as an animal-free, biocompatible substitute for gelatin. Uniquely, the polypeptide allows the independent controlling of the melting temperature of the gel, the stiffness and crosslink density of the gel, the lower critical gel-forming concentration related to the length of the molecules, and unrelated (e.g. biological, physiological, pharmaceutical or signalling) functions of the molecule.

## Description

The invention pertains to a block co-polypeptide, notably an artificial collagenous polypeptide. In particular, the invention pertains to an artificial collagenous block co-polypeptide capable of forming a hydrogel, as gelatin does. The invention also pertains to uses of artificial collagenous block copolypeptide, notably in medical applications such as in surgical materials, drug delivery or tissue engineering.

Natural gelatin is regularly obtained by heat denaturation, i.e. a disintegration of the triple helix structure, after hydrolysis of the intermolecular covalent crosslinks in fibrillar collagen from animal sources, notably type I and type III collagen from bovine or porcine origin. Collagen is made up of three polypeptide strands. Each of these is a left-handed extended helix, and together they are twisted into a triple helix. Animal collagen is held together by intermolecular crosslinks that are formed after formation of the collagen molecules themselves and their organisation into fibrils, the degree of crosslinking increasing with the age of the tissue. These crosslinks must be largely hydrolyzed in order to make the collagen and - after heat denaturation - the resulting gelatin, soluble. However, the necessary hydrolysis, brought about at extreme pH, also results in unwanted and largely uncontrollable partial hydrolysis of the collagen backbone, and (when treated at high pH) in deamidation and shifting of the isoelectric point.

Gelatin is not only widely used as a food ingredient, as drug capsule material, as indispensible ingredient in photographic prints and (traditionally) photographic films, but also as an important biomedical material (reflecting an annual kiloton material or billion dollar turnover). It is for example used in bone implants, retinal implants, tissue enhancement, in vascular prosthesis, sponge embolization therapy, as arterial or stanching plugs, as scaffold upon which to seed cells for tissue engineering applications, as ingredient of medical glues, in wound- and burn-dressings, as a carrier material for controlled drug delivery, as a colloidal biocompatible polymer in blood supplementation fluids and in vaccines (the latter two applications involving low-viscous, non-gelling gelatins).

In recent years, natural gelatin has become associated with drawbacks. Particularly the animal origin is seen as a risk, in view of the chances that the animal collagen may carry transmissible disease agents, e.g. spongiform encephalopathy prions, such as the well-known bovine spongiform encephalopathy (BSE), which is seriously suspected to be linked to the fatal neurological disease of Creutzfeldt-Jakob in humans. In food, and even more strongly in pharmaceutical applications, this is a critical issue, which has resulted in regulatory restrictions on the origin of the gelatins used in these products.

Thus, it is desirable to be able to avoid animal collagen as a source for gelatin. Additionally, a drawback to gelatin of animal origin is its highly complex and variable composition, depending on the heterogeneous and unknown genetic origin and age of the animals (in older animals, the degree of crosslinking gradually increases), on the conditions under which they were raised, etc.. The regular processes of extracting gelatin from animal hides and bones results in a heterogeneous mixture of proteins comprising polypeptides of various chain-length, degrees of crosslinking / branching, isoelectric points, local amino acid composition and sequence and helix-forming propensity. From an economical point of view in various industries, and from the point of view of a desired predictability of properties particularly in pharmaceutical applications, it is desirable to provide gelatin of a more precisely predictable, preferably tunable structure.

To this end, recombinant gelatins have been proposed, such as in US 6,992,172. Essentially a desired type of collagen, or part of it, or concatemers of (part of) that collagen, is produced by recombinant techniques, and the recombinant molecule is extracted.

Recombinant gelatins produced in cells like yeast and other microbial cells naturally devoid of peptidyl-prolyl-4-hydroxylase are non-gelling. Gel formation is induced in sufficiently long gelatin molecules (generally well over 10 kDa) by specific hydroxylation of proline in specifically the Yaa-position of the repeating (Gly- Xaa- Yaa) amino acid triplet sequence of gelatin, to yield 4-hydroxy proline. In animals, this occurs post-translationally by an enzyme (peptidyl-prolyl-4-hydroxylase) that is generally lacking in microbial hosts used for making the recombinant gelatin.

In the present description, which relates to molecules with a repeating amino acid triplet sequence composed of either consistent (Gly- Xaa-Yaa), consistent (Yaa - Gly- Xaa), or consistent (Xaa- Yaa - Gly) repeats, the unspecified amino acids just C-terminal and just N-terminal of Gly are denoted "Xaa and Yaa", respectively.

In traditional gelatins, there is no single, fixed and invariant melting temperature, because the distribution of effective helix melting temperatures in the gel is dependent on many parameters including the molecular composition and the thermal history, as the gel structure evolves slowly in time during weeks to even months. The Tm can therefore not be precisely controlled. The same is true for the distance between the non-covalent triple helical crosslinks or mesh width and for the gel structure. Moreover, the structure of traditional gelatin gels is only hardly understood and is confined to some general ideas, which makes it more difficult to engineer novel properties. After fast cooling, the gel consists of a relatively large number of small thermolabile helices connected by relatively long single strands. As the helices grow and the helix content increases in time, and especially after gradual cooling, the gel becomes populated with larger and more thermostable helices connected by ever shorter single strands. It is not precisely clear how the average distance between the non-covalent triple helical crosslinks and the broadness of the distribution of these distances evolves, but it is certain that the structure and the distribution of distances between crosslinks in the gel is in this way related to the average thermostability and the distribution of helix melting temperatures in the gel. Thus, the structural and thermal parameters are interrelated and cannot be uncoupled. Moreover, due to this complexity, they are hardly accessible to functional precision engineering. Another factor hampering the precision engineering of traditional gelatin functionality is the difficulty to control the molecular composition, due to the above-mentioned complexity of the composition and the varying origin of the collagen source, although some control may in principle be exerted by selecting the animals (genetic background, age, raising conditions), which implies additional costs, or by using for example fish collagen with a lower proline and hydroxyproline content and lower melting temperature. A reference in this respect is De Wolf, F. A. (author & chapter-editor) et al. (2003) Chapter V - Collagen and gelatin. In: Industrial Proteins in Perspective (Aalbersberg, W. IJ. et al.,eds.), Progress in Biotechnology 23, Elsevier Science, Amsterdam, pp. 133-218.

Better control is possible by recurring to recombinant production. Selected collagen genes or gene segments from various species and even designer gelatins can be expressed in suitable heterologous expression systems, for production of unimolecular, monodisperse gelatins with a selected and defined molecular weight, isoelectric point, amino acid sequence, etc. However, most recombinant expression systems like microorganisms and plants do not naturally perform the above-mentioned sequence-specific prolyl-4-hydroxylation required for the formation of thermostable triple helices, and thus, they are not naturally suitable to produce gel forming gelatins.

This can be remediated by co-expression of suitable (e.g. animal or human) peptidyl-prolyl-4-hydroxylase (P4H), but this normally requires the balanced expression of two additional genes coding for the B and the catalytic a subunit of the animal ααββ tetrameric enzyme, which becomes unstable when sufficient collagen substrate is lacking and can fall apart, leading to irreversible α subunit aggregation. Moreover, as the β subunit has additional functions and is equal to the protein disulfide isomerase, it is always possible that unbalanced expression interferes with other cellular processes. Notwithstanding these draw-backs, coexpression of the P4H with natural collagen or collagen segments (gelatins) has been successfully obtained by others, leading to homogeneous hydroxylation of the prolines in the Yaa-positions of the repeating (Gly- Xaa - Yaa) amino acid triplet sequence of the entire collagen or gelatin molecule and to partially or fully trimeric product. A drawback of this approach, as reported in the art, is that it results in intracellular trimer formation, and secretion of the trimeric product appears to be precluded[Vuorela et al., EMBO J. 16:6702-6712 (1997); Myllyharju et al., Biochem. Soc. Trans. 28:353-357 (2000); Nokelainen et al., Yeast 18:797-806 (2001); Olsen et al., J. Biol. Chem. 276:24038-24043 (2001); Pakkanen et al., J. Biotechnol. 123:248-256 (2006)] , even in the expression system in which the highest levels of secreted non-hydroxylated gelatin were reached: the yeast *Pichia pastoris* [Werten et al., Yeast 15:1087-1096 (1999)].

However, with none of the above methods it is possible to precisely engineer the melting temperature or the distance between trimer-forming (nonvalent crosslink-forming) domains in the gel, as a determinant for the effective mesh width and gel stiffness, let alone that it would be possible to engineer both parameters independently from each other, or independently from the molecular size.

It is desired, e.g. for applications in drug delivery, to control, preferably precisely, the melting temperature and the distance between the non-covalent triple helical crosslinks of the gel, independently of each other. This means that if, e.g., to fit a certain (protein) drug in a gelatin hydrogel matrix a large distance between the non-covalent triple helical crosslinks is desired, such could be combined with retaining a hydrogel at human body temperature. For drug delivery purposes one would like to be able not only to choose (adapt) the distance between the non-covalent triple helical crosslinks to the size of the component to be released, and to the intended release rate, but independently from the distance between the non-covalent triple helical crosslinks, to choose if the gel should melt upon introduction in the body (room temperature < Tm < body temperature), or if it should stay a gel at all times, or if it should only start melting in tissues with a higher temperature (e.g. inflammated or externally heated tissue). The minimal concentration at which a gelatin network (gel) is formed is determined by the molecular size as well as by the number of trimer-forming (noncovalent crosslink forming) domains per chain. On the other hand, the stiffness of the gel and the permeability to drugs, in other words the porosity or the mesh width of the gel, is determined mainly by the number of trimer-forming (noncovalent crosslink-forming) domains per chain, the length of such domains, the mutual distance - along the chain - between the trimeric domains, and the polypeptide concentration. Depending on the application and the procedure used to prepare gels and load these with smaller and larger drugs at high or low viscosity, and before or after setting of the gel, it would be desirable to engineer the minimal protein concentration required for gel formation and the intended distance between the non-covalent triple helical crosslinks and stiffness of the gel (in other words the chain length and the number and length of trimer-forming domains) independently from each other. However, such possibilities are not offered by the traditional animal-derived or newer recombinant gelatin types available to date.

For surgical purposes, bone implants, retinal implants, tissue enhancement , in vascular prosthesis, sponge embolization therapy, as scaffold upon which to seed cells for tissue engineering applications, as ingredient of medical glues, in wound- and burn-dressings, it is highly desirable to engineer the stiffness of the gel, which is dependent on the distance between the non-covalent triple helical crosslinks and the concentration of these crosslinks in the gel. Moreover, it is desirable to be able do so without changing (i.e. independently of) the melting temperature, which should always be independently optimized for the application concerned. Finally, for similar reasons as indicated above in relation to drug delivery applications, it may be desirable to be able to engineer noncovalent crosslink density and molecular size (chain length) independently from each other. However such possibilities are not offered by the traditional animal-derived or newer recombinant gelatin types available to date.

The present invention now provides an artifical gelatin that, if desired, can be made without the aforementioned proline hydroxylation step. Also, a more versatile gelatin can be provided, allowing the incorporation of many desired structural moieties. Furthermore, the gelatin's melting temperature, molecular size and distance between the non-covalent triple helical crosslinks can be independently controlled.

In order to better address the foregoing objectives, the invention provides a multiblock polypeptide (artificial gelatin) comprising a number of relatively short (for example 6 to 90 amino acids and preferably 9 to 60 amino acids) thermoreversible trimeric knot-forming blocks ("Trimerizing Blocks" hereinafter denoted "T"), alternating with blocks acting as spacers between the trimeric knots of the network ("Spacer Blocks" denoted "S"). These Spacer Blocks S can be of various nature and can be relatively long (for example 50 to 3000 amino acids, preferably 200 to about 1000 amino acids).

Thus, the invention, in one embodiment, is a block copolypeptide comprising at least two Trimerizing Blocks T and at least one Spacer Block S, with blocks T and S alternating, wherein each T independently denotes a Trimerizing Block being a polypeptide block capable of forming a thermoreversible collagen-like triple helix structure, and wherein each S independently denotes a Spacer Block being a polypeptide not intrinsically capable of forming a triple helix structure. Preferably, the Spacer Blocks do not substantially participate in any supramolecular structure. For example, the Spacer Blocks S could have a gelatin-like or even nature-identical collagen-derived amino acid sequence devoid of hydroxyproline, or else they could have a gelatin-like or nature-identical collagen-derived amino acid composition, optionally comprising hydroxyproline, in combination with a positioning of glycine residues that is not consistent with any repeating amino acid triplet sequence (Gly-Xaa-Yaa)n, (Yaa-Gly-Xaa)n, or (Xaa-Yaa-Gly)n. Either of the latter two designs promotes the Spacer Blocks S to assume a random or unordered conformation)

Without wishing to be bound by theory, the present inventors believe that the foregoing block polypeptide provides a sound basis for making precisely structured gels. The blocks capable of forming a triple helix serve as knots required to make a more or less continuous three-dimensional network. These blocks determine the collagenous nature of the block co-polypeptide of the invention. The random coil blocks assuming an unstructured (random) conformation structure act as spacers and can be made of desired length, and can be provided with desired functionality, provided that, most preferably, hardly or no triple helix- or other supramolecular structure formation can occur in the Spacer Blocks S. The main parameter affecting melting temperature is the length of the triple helix forming blocks. The main parameter determining the distance between the non-covalent triple helical crosslinks in a gel formed by the block copolymer, is the length of the Spacer Blocks S. It should be clear that, where such would be desirable, additional alternating T and S blocks, and even an alternating series of different kinds of trimer forming and non-trimer-forming blocks can be added to the chain (i.e. can be incorporated in the gelatin molecule), according to the same principle and according to very similar procedures. Also, it should be clear that the non-trimer-forming blocks can be chosen to comprise non-hydroxylated nature-identical (for example human-identical) collagen segments with sufficiently low helix thermostability, or other natural non-trimer-forming protein domains with the amino acid sequence naturally occurring in those proteins, each individual domain as well as their mutual order being precisely engineered (precisely as intended), in contrast to random copolymers or polymers with random block length or block order.

This concept opens up the possibility of generating a series of animal-free and biocompatible gels for broad medical and pharmaceutical application, with independently tunable crosslink density, chain length, melting temperature and other properties. In favorable contrast to traditional gelatin, the dynamic elasticity of the new material, in which it is possible to have only one single (well-defined) type of cross links formed, is independent of the thermal history of the gel (i.e. the thermal history below Tm). Also in favorable contrast to traditional gelatin, melting temperatures above 30 °C and even above 40 °C now become feasible. Such gels will not melt in the (healthy) body, but could specifically be made to melt in case of fever, or when certain tissues are artificially heated by external heat or radiation sources. In certain applications, this will obviate the need to covalently crosslink the gel (e.g. by chemical means) before it is introduced into the body. This allows for melting of the gel in the body at command, thereby increasing the biodegradability and biocompatibility, or, conversely, to introduce a molten gel that will gel once at body temperature. These features extend the application potential and versatility of the gelatin for pharmaceutical, surgery, wound treatment and regenerative medicine / tissue engineering applications as well as cell culture and other laboratory applications. Similar advantages may be pertinent to other applications.

### Trimerizing Blocks

The Trimerizing Blocks T each independently denote a polypeptide block capable of forming a triple helix structure. Thus, three polypeptide chains will be able to be trimerized into a triple helix, so as to result in knots needed to obtain a gel.

The triple helix-forming blocks are comprised of such repeating units that, except for chain-ends, every third amino acid is an amino acid capable of fitting into the core of the triple helix. Most preferably, as in collagen, this is the smallest natural amino acid, viz. glycine, but recent science indicates that also D-Ala and D-Ser have this capability of fitting into the core of the triple-helix. The triple helix forming block will be further described hereinafter with reference to glycine, but it will be apparent that analogous structures in which e.g. D-Ala is substituted for glycine, are within the scope of the invention. Glycine is preferred, as it does not require chemical synthesis to be built in, as D-amino acids do.

The triple helix-forming blocks (i.e. Trimerizing Blocks), each independently, comprise repeating units selected from the group consisting of (Xaa-Yaa-Gly), (Yaa-Gly-Xaa), and (Gly-Xaa-Yaa), wherein Xaa and Yaa stands for unspecified amino acid, provided that the selection is consistent in that every third amino acid in the chain is consistently a glycine.

The term "repeating units" expressly includes a plurality of units that do not each have identical amino acids, but in which the amino acids in different units (apart from the repeating glycine) may be different.

The choice of amino acids in the above-mentioned Xaa and Yaa positions (i.e. outside the canonical Gly-positions) is not entirely free, as not all amino acids will lead to the formation of a sufficiently stable triple helix. The triple helix forming abilities of amino acid triplets have been well studied. Reference is made to Persikov et al (2005) J Biol Chem 280 (19): 19343-349. Herein suitable guidance on triplet stability is given by an indication of triple helix melting points, see Table 1 on page 19345 therein. In the present invention, those triplets can generally be- chosen for which a melting temperature of 36°C or higher is indicated in said table.

It is preferred for the triplets in the triple helix forming blocks to be selected from the group consisting of GPR, GPP, GPM, GPI, GPQ, GEP, GPA, GER, GPV, GPE, GQR, GKR, GPT, GKP, GKQ, GRP, GQP, GDP, GEM, GAR, GRR, and mixtures thereof. The letters used are in accordance with the conventional one-letter nomenclature for amino acids. G is glycine, P is proline, R is arginine, M is methionine, I is isoleucine, Q is glutamine, E is glutamic acid, D is aspartic acid, A is alanine, V is valine, T is threonine, K is lysine. The foregoing is written with the unit structure [Gly- Xaa - Yaa] in mind, but will be similarly applicable to unit structures [Yaa -Gly- Xaa] and [Xaa - Yaa -Gly], provided that the selection is consistent in that every third amino acid in the chain is consistently a glycine.

Thus, in the block co-polypeptide of the invention, the Trimerizing Block preferably is built up of amino acid triplets selected from the group consisting of (Gly- Xaa - Yaa), (Yaa -Gly- Xaa), and (Xaa - Yaa -Gly), wherein Xaa and Yaa are unspecified amino acids, to the extent that the sequence (Xaa - Yaa), resulting from said triplets, is selected from the group consisting PR, PP, PM, PI, PQ, EP, PA, ER, PV, PE, QR, KR, PT, KP, KQ, RP, QP, DP, EM, AR, RR and mixtures thereof.

In addition, a triple helix forming block can also comprise combinations of two triplets that are capable of forming an electrostatic complex. These double triplets are preferably selected from the group consisting of GPR-GEP, GPK-GEP, GPE-GKP, and GPE-GRP, GPR-GDP, GPK-GDP, GPD-GKP, and GPD-GRP and in these cases the amino acid order is critical. In this case mixtures are possible only if in subsequent double triplets the initial charge in any double triplet is the opposite of the terminal charge of the preceding double triplet.

Further, although the invention advantageously makes it possible to avoid proline hydroxylation, the present invention can also be carried out by including, as the triple helix forming blocks, amino acid triplets that can be rendered into a stable triple helix by proline hydroxylation. For these triplets the order [Gly-Xaa-Pro] is relevant, and these are preferably selected from the group consisting of GPP, GAP, GKP, GRP, GQP, GDP, GLP, GVP, GMP, GIP, GNP, GSP, and mixtures thereof.

Most preferably, the triple helix forming blocks are proline rich, even up to a proline content of 67% (all amino acids other than glycine are proline, i.e. [Gly-Pro-Pro], [Pro-Gly-Pro], or [Pro-Pro-Gly], provided that the selection is consistent in that every third amino acid in the chain is consistently a glycine.

The triple helix forming blocks generally have a length of 6 to 90 amino acids and preferably 9 to 60 amino acids and more preferably of from 18-48 amino acids.

The length and the amino acid composition of the triple helix-forming block will largely determine the melting temperature of the polypeptide of the invention. Thus, a melting temperature can basically be set irrespective of the distance between the non-covalent triple helical crosslinks of the gel, because this distance is determined by the length of the Spacer Blocks S, situated between the Trimerizing Blocks T). By varying the amino acid composition of the Trimerizing Block T there is also considerable freedom to obtain a desired melting temperature independently, within limits, of the size of these knot forming regions.

As indicated above, the block co-polypeptide of the invention minimally comprises two Trimerizing Blocks T alternating with one Spacer Block S. It is also possible, within the present invention, to provide a larger number of alternating T and S blocks.

An advantage of including more than two Trimerizing Blocks per polypeptide molecule is that therewith the crosslink density and the modulus of elasticity can be controlled independently of the length of the molecule and independently of the distance between the two outer Trimerizing Blocks T in the molecule

A particular advantage can be obtained by selecting different blocks "T", having different properties such as melting point. Thus, e.g., certain pairs or larger sets (groups) of blocks T within the co-polypeptide structure can be selected with a (slightly) lower melting point than other pairs or sets of Trimerizing Blocks T. This can be of advantage for various applications, in which it is desired to enlarge the distance between the non-covalent triple helical crosslinks *in situ* (by selective melting of only certain pairs or sets of trimer helices) whilst a gel structure is maintained by the presence of trimer helices with a higher melting temperature that are not affected at the chosen temperature. This can be of use, e.g. in the controlled and sustained delivery of drugs: a relatively tight gel structure comprising the drug will be administered, and after melting of some trimer helices the resulting larger distance between the non-covalent triple helical crosslinks will lead to drug release, still in a. controlled fashion by virtue of the remaining basic gel structure. Also, e.g. a wound or injury-covering material can be provided that, after melting of the internal trimer helices, will release a drug or a healing agent, with the basic gel structure (determined by the terminal trimer helices) that covers the wound or injury in the first place, will remain.

It should be noted that the boundaries of the T blocks may vary with a length of a few (generally 1-5) amino acids. E.g., the exact starting point of the trimer helix formation in a terminal block T need not be the first amino acid, in which case the terminal amino acids just extend outside of the trimer helix. Similarly, the transition from trimer helix to random coil may go gradually, i.e. with a widened end of the helix extending into the "random coil" structure.

In co-polypeptides of the invention comprising more than the two terminal T blocks, each block will preferably have a length within the aforementioned boundaries. In these cases it is possible that the total size of the molecule is similar to that in a simple three-block co-polypeptide TST, and that the lengths of the S blocks are reduced to accommodate more T blocks.

### Spacer Blocks

The term "Spacer Block" S as used herein denotes a polypeptide block that is not intrinsically capable of forming a triple helix structure. I.e., viewed in isolation, a block that is capable of forming a trimer, is a Trimerizing Block, and a block that is not capable of forming a trimer is a Spacer Block.

This property of intrinsically not forming trimers, does not exclude that a portion of an Spacer Block could participate in the formation of trimers under the influence of one or more Trimerizing Blocks. E.g., in the event of an Spacer Block that comprises triplets of a composition not intrinsically capable of forming a stable trimer, but having a consistent repetition of glycine every third amino acid, a portion of such an Spacer Block adjacent to a Trimerizing Block could nevertheless be stabilized into a triple helix structure, by virtue of the adjacent Trimerizing Block trimers. Also, again in the event of a Spacer Block with a non-trimerizing composition but having a consistent repetition of glycine every third amino acid, a portion of one of such Spacer Blocks could form a trimer with two Trimerizing Blocks, or two of such Spacer Blocks with one Trimerizing Block.

To the extent that a portion of an Spacer Block were intrinsically capable of forming a triple helix structure, it will be understood that such a portion of an Spacer Block is in fact a further Trimerizing Block, i.e. the Spacer Block will then not in fact be regarded as a single Spacer Block, but will be regarded an S-T-S chain.

Preferably, the S-blocks do not substantially form any supramolecular structures and are to be considered as a random coil. The term "random coil" as used herein, is to be interpreted broadly as referring to any unordered or, as the case may be, unfolded structure rather than a secondary structure such as an alpha helix or a beta sheet.

It is possible for one or more of the Spacer Blocks S, each independently, to comprise amino acid triplets promoting the formation of collagen-like triple helical structures, as long as said triplets occur in amounts low enough to prevent the formation of collagen-like triple helical structures.

It is also possible to suppress the formation of collagen-like triple helical structures in one or more of the Spacer Blocks S, each independently, by avoiding of the occurrence of glycine in most of those positions that are separated from another glycine by two intermediate residues in the amino acid sequence of the Spacer Blocks S concerned, irrespective of the amino acid composition of the Spacer Blocks S concerned. I.e., the occurrence of glycine consistently in every third position it thereby avoided. Thus, the Spacer Block S may in fact have a composition that would have resulted in the formation of collagen-like triple helical structures, had glycine consistently occurred in every third position.

Besides the foregoing, the selection of amino acids for the Spacer Block is essentially free, be it that in order to form a hydrogel, the mid block should be water soluble and thus should not contain too many hydrophobic residues. This principle may be adapted if gels in solvents like ethanol are to be made.

With the aforementioned Trimerizing Blocks capable of forming knots, it will be apparent that the Spacer Blocks S act as spacers so as to determine the distance between the non-covalent triple helical crosslinks of the gel.

Preferably the Trimerizing Blocks T are short for two reasons. Short Trimerizing Blocks T will result in a more precisely defined network structure with fewer possibilities for mutual misalignment of the three chains that take part in a trimeric helix, and with fewer possibilities for incomplete helix formation (half folded helices). Short blocks T will also speed up the completion of helix formation and the attainment of steady gel properties. In relation to this, the Spacer Blocks S are preferably much larger than the Trimerizing Blocks T, as relatively long blocks, as spacers between the trimeric knots, will enhance the uptake capacity for water and active pharmaceutical ingredients and the flexibility of the gel. On the other hand, if a relatively high modulus of elasticity is desired, shorter spacers can be chosen. The Spacer Blocks S generally has a length of 50 to 3000 amino acids, preferably 200 to about 1200 amino acids

The person skilled in the art is aware of polypeptide moieties that are capable of forming secondary structures, and also of how to avoid this. Many specific polypeptide domains can be selected that intrinsically do not form secondary structures. Also, specific amino acids can be incorporated that serve to counteract secondary structure formation. This, e.g., holds for proline provided that it is not present in too high a concentration in order to prevent the formation of collagen-like triple helix structures. For example (Gly- Xaa-Yaa), (Xaa - Yaa -Gly), or (Yaa -Gly- Xaa) repeats with a content of not more than 20 -25 % non-hydroxylated proline and not too many other collagen-helix-promoting amino acids (such as indicated above) will generally not form triple helices, while the formation of alpha helices and beta sheets is in general efficiently suppressed. And if proline is present at a higher concentration, such that in a consistent triplet structure with glycine in every third position it would start to give rise to the formation of collagen-like triple helix structures, such triple helix formation can be suppressed by avoiding such a consistent triplet structure with glycine in every third position. For example, the triplet GPP, if used as a repeating unit, will form a triple helix. If, with the same amino acids, a random polypeptide is made, i.e. by avoiding the repetition of G every third amino acid, a "random coil", i.e. an unordered or unfolded conformation, will result. Similarly, triplets that require hydroxylation to form stable triple helices, can be used in the Spacer Blocks S in the unhydroxylated form.

In fact, so as to avoid, depending on its-intended function, undesired secondary structures in the Spacer Blocks S, it is preferred to include throughout the Spacer Blocks S at least 5% (mol/mol) of proline, preferably at least 10% (mol/mol) and more preferably at least 20% (mol/mol). E.g. a preferred range is some 10-25% (mol/mol) of proline in the Spacer Blocks S, as this will contribute to avoiding secondary structure formation. It is most preferred to combine this with triple helix forming blocks in which 50-100% of the non-glycine amino acids is proline, with most preferably all non-glycine amino acids being proline (in which case the triple helix block thus has 67% of proline).

Another way of preventing secondary structures is to ensure net (excess) charge. E.g., whilst poly(Gly-Ala) tends to form beta sheets or other crystalline secondary structures, poly((Gly-Ala)3-Gly-Glu) dissolves well at neutral to alkaline pH when the carboxyl groups are charged, and would be suitable as a random-coil block. As the person skilled in the art will appreciate, many other possibilities exist to obtain unordered or unfolded structures by ensuring a net charge.

The Spacer Blocks S offers a great variety of possibilities to include desired polypeptide structures, provided that they do not interfere with the structure of the gel. E.g. peptide domains that bind to animal cell integrins or other cell surface structures, so as to enable these to adhere to the gelatin of the invention, growth factor or growth factor-mimicking domains, antimicrobial sequences and other bioactive or biological signal-representing sequences, domains that interact with active agents, such as drugs included in the gelatin (e.g. for transportation, stabilization, controlled release, etc.), or polypeptide domains that serve as ligands for biological targets, such as - but not limited to - single-chain antibody domains (the latter serves to facilitate local drug delivery of a drug also included in the gelatin). Also, one could incorporate sequences that specifically interact with the medicinal protein and can be provided with an external prompt to counteract the interaction, resulting in release of the protein (e.g. based on charge effects). Provided that these functional domains inserted into the "random coil" blocks will not form non-covalent crosslinks in the gel (e.g. coiled coils, beta-sheet stacks, etc.) these functional / bio-active domains do not need to have an unordered structure themselves, but could e.g. assume alpha-helical or beta-sheet conformations.

### Manufacture of the polypeptide

The polypeptide can be manufactured by means of standard methods available in the art.

Although not entirely excluded, it is hardly possible to prepare chemically a synthetic polypeptide of the invention, or to build the full polypeptide by joining synthetic parts. It is preferred to use recombinant technologies because this allows to produce long and monodisperse polypeptides with a defined sequence order.

Polypeptide manufacture through recombinant technologies is known. In general an artificial DNA sequence is provided in a known manner, having a sequence encoding for the desired polypeptide. Various cloning methods are nowadays available for the construction of G-C-rich, repetitive DNA sequences and genes of interest (Padgett K.A. & Sorge J.A. (1996) Gene 168: 31-35; McMillan R.A., Lee T.A.T. & Conticello V.P. (1999) Macromolecules 32: 3643-3648; Werten M.W.T., Wisselink W.H., Jansen-van den Bosch T.J., de Bruin E.C. & de Wolf F.A. (2001) Protein Engineering 14(6): 447-454; Goeden-Wood N.L., Conticello V.P., Muller S.J. & Keasling J.D. (2002) Biomacromolecules 3: 874-879; Won J.-I. & Barron A.E. (2002) Macromolecules 35: 8281-8287; Henderson D.B., Davis R.M., Ducker W.A., Van Cott K.E. (2005) Biomacromolecules 6: 1912-1920; Lu Q. (2005) Trends in Biotechnol. 23(4): 199-207; Mi L. (2006) Biomacromolecules 7: 2099-2107). This does not require elucidation here. The DNA, in a suitable vector, is expressed in host cell. Suitable hosts are e.g. *Picha pastoris, Hansenula polymorpha*, *Kluyveromyces marxianus var. Lactic, Aspergillus niger* / *oryzae* / *sojae* /*awamori, Bacillus megaterium* / *brevis* / *subtilis* / *thuringiensis, E. coli K12 derivative*. The preferred host is *Pichia pastoris* and the preferred mode of expression is secretion into the extracellular medium. In the present invention the latter is achieved, even though the block copolypeptide is capable of forming collagen-like triple helices and hydrogels.

When making the block co-polypeptides according to the invention, the length of the Trimerizing Blocks T, at given amino acid compositions and sequences of the Trimerizing Blocks T, can be tuned, each independently, so as to tune the melting temperature of said Trimerizing Blocks. I.e., in the event of a given amino acid composition for the T block, increasing the length of the Trimerizing Block, will lead to an increase of the melting temperature thereof, and decreasing the length of the Trimerizing Block will lead to a decrease of the melting temperature thereof.

### Gel formation

The block co-polypeptide of the invention will generally be obtained in the form of a lyophilized suspension. In order to avoid gel formation during purification (isolation) of the product from the fermentation broth, it may be preferred in some cases to include a heating step so as to melt possible gel structures formed while the protein is in suspension.

Just as natural gelatin, the block co-polypeptide of the invention is capable of forming a gel upon cooling heated solutions. The temperature at which a gel is formed can be tuned, by varying the length of the triple-helix forming blocks as outlined above. Generally this temperature will vary from 10 to 60 °C, preferably from 15 to 50 °C, but a larger range is also possible.

### Uses of the polypeptide

The present invention uniquely provides an animal-free, biocompatible polypeptide in which several parameters that affect possible uses, can be controlled independently of each other. This refers to the melting temperature, the modulus of elasticity (linked to mesh width. pore size and distance between knots), the molecular length (linked to the minimum gel concentration), and the optional presence of bioactive and ligand binding sequences in the Spacer Blocks S. This type of independence of controllable properties neither exists in natural gelatins, nor in current monodisperse recombinant gelatins.

Various uses of the block copolypeptide gelatins of the present invention are contemplated, including the above-mentioned applications of gelatin and related applications. E.g. bone, retinal, tissue-enhancing and other implants, in vascular prosthesis, sponge embolization therapy, device coatings, scaffolds for tissue and cell culture, medical glues, arterial or stanching plugs blood supplementation fluids, drug carriers, wound dressings and vaccines. In particular, the present invention comprises encapsulants, stabilizing agents, film-forming agents, moisturizing agents, emulsifiers, thickening agents, gelling agents, colloidal agents, adhesive agents, flocculating agents, and refining agents comprising block copolypeptide gelatin.

The present invention provides in one embodiment a pharmaceutical composition comprising block copolypeptide gelatin. In specific embodiments, the present invention provides a hard gel capsule, a soft gel capsule, a tablet coating, a plasma expander, a colloidal volume replacement material, an injectable (or otherwise administrable) tissue enhancer, e.g. in plastic or cosmetic surgery, a graft coating, a medical sponge, a medical plug, a moisturizing and protective sheet (e.g. to cover burns), a gel-spun resorbable surgical thread (yarn), a pharmaceutical stabilizer, and a microcarrier comprising block polypeptide gelatin. In one aspect, the present invention encompasses a kit comprising a composition comprising block polypeptide gelatin, and a device for delivering the composition to a subject.

An edible composition comprising block polypeptide gelatin is also contemplated, as are protein supplements, fat substitutes, nutritional supplements, edible coatings, and various microencapsulants comprising block polypeptide gelatin.

Photographic compositions comprising block polypeptide gelatin are also contemplated.

In other embodiments, the invention encompasses a cosmetic composition comprising block polypeptide gelatin, an industrial composition comprising block polypeptide gelatin, a cell culture composition comprising block polypeptide gelatin, and a composition for laboratory use comprising block polypeptide gelatin

In general, embodiments in which block polypeptide gelatin is partially or fully hydroxylated to expand the range of melting temperatures to higher values and to speed up the rate of gel formation, are also contemplated.

The invention will be illustrated hereinafter with reference to the following non-limiting Examples and Figures.
Figure 1
   Graphic representations of an example of blockcopolypeptide and hydrogel.
   (Top): Schematic representation of the triblock structure of the TP₄T and TR₄T copolymers discussed in Example 1. Monomeric T-type Trimerizing Blocks T are located at either end of the molecule; large circle enveloping coiled line: 4-or R4-type Spacer Blocks S, which assume unstructured ('random') conformation, as the central block of the triblock molecule.
   (Middle): Schematic representation of the formation of triple helical knots by three T blocks located at the termini of the above-described TP₄T and TR₄T copolymers.
   (Lower right): Schematic representation of a small part of a gel formed by the above-described TP₄T and TR₄T copolymers. Small rectangles: triple helical knots formed by three T blocks located at the termini of the TP₄T and TR₄T copolymers. Circles: P4- or R4-type Spacer Blocks S forming the middle part of the TP₄T and TR₄T copolymers. These Spacer Blocks assume unstructured ('random') conformation
Figure 2
   Production of purified gelforming co-polypeptides. (a) SDS-PAGE. Lane M: protein marker, lane 1: TP₄T, lane 2: TP₈T, lane 3: TP₁₂T, lane 4: TR_{4T}, lane 5: TR₈T. (b) MALDI-TOF of purified TP₄T and TR₄T.
Figure 3
   Graph depicting lysozyme release (vertical axis) vs time (horizontal axis) as described in Example 1.
Figure 4
   Table of oligonucleotide sequences as referred to in Example 1.

### Example 1

This example refers to the production of specific T-S-T triblock copolypeptides, denoted TPₙT and TRₙT, which comprise one central Spacer Block S and two Trimerizing Blocks T positioned at either end of the triblock polypeptide. Herein, the Trimerizing end Blocks T comprise an uninterrupted (Pro-Gly-Pro)₉ sequence with a length of about 8 nm in triple helix conformation and the central Spacer Block S consists of a concatemer (i.e. an n-fold repeat) of either a "P"-type or "R"-type block, each with a gelatin-like amino acid composition, a length of about 100 amino acids and an amino acid sequence as specified below. Both P and-R blocks assume an essentially unordered (i.e. random) conformation with a tentatively estimated end-to-end distance in relaxed form (i.e. without any externally applied mechanical force) of about 10-15 nm for n=1, about 20-25 nm for n=4, about 30-35 nm for n=8, and about 40 nm for n=12). Thus, the molecular lay-out of the molecule is a TPₙT or TRₙT triblock structure, with n being an integer, as specified below.

### METHODS

### Construction of TP₄T

A *P. pastoris* vector for secreted expression of TP₄T was constructed as follows. The T block was prepared by PCR using the oligonucleotides T-FW and T-RV (Table 1). The ∼0.1 kb product was cloned into vector pCR4-TOPO (Invitrogen), resulting in vector pCR₄-TOPO-T. The vector pMTL23-P₄ (Werten M.W.T., Wisselink W.H., Jansen-van den Bosch T.J., de Bruin E.C. & de Wolf F.A. (2001) Protein Engineering 14(6): 447-454) contains a gene encoding the custom-designed, highly hydrophilic 36.8 kDa collagenous protein 'P₄'. This vector was digested with *Dra*III (5' to the P₄ gene) and dephosphorylated. Vector pCR4-TOPO-T was digested with *Dra*III/*Van*91I. The released T block was ligated into the linearized and dephosporylated vector, to yield vector pMTL23-TP₄. This vector was then digested with *Van*91I (3' to the P₄ gene) and dephosphorylated, and again a *Dra*III/*Van*91I digested T block was inserted, to yield vector pMTL23-TP₄T. The TP₄T fragment was released by digestion with *Xho*I/*Eco*RI and ligated into the correspondingly digested *P. pastoris* expression vector pPIC9, to yield vector pPIC9-TP4T.

### Construction of R₄ and TR₄T

The gene encoding R₄ was constructed by concatenating four copies of an R gene monomer. The monomeric gene was designed by randomizing the sequence of the P gene monomer in such a way that not every third residue is glycine, preventing the formation of collagen triple-helices by the encoded protein while maintaining the same amino acid composition. The R gene monomer was constructed by overlap extension PCR using oligonucleotides OVL-RA-FW and OVL-RA-RV for the 5' half of the gene, and oligonucleotides OVL-RB-FW and OVL-RB-RV for the 3' half (for oligonucleotide sequences see Table 1). The products of these reactions were combined by overlap extension PCR to generate the entire R gene monomer. The monomer was cloned into vector pMTL23 via *Xho*I/*Eco*RI*,* and multimerized as knownfor P₄ (Werten M.W.T., Wisselink W.H., Jansen-van den Bosch T.J., de Bruin E.C. & de Wolf F.A. (2001) Protein Engineering 14(6): 447-454), resulting in vector pMTL23-R₄. The R₄ fragment was released by digestion with *Xho*I/*Eco*RI and ligated into the correspondingly digested *P. pastoris* expression vector pPIC9, to yield vector pPIC9-R₄. The construction of pMTL23-TR₄T and subsequent subcloning into vector pPIC9 was analogous to the procedures described for pPIC9-TP₄T.

### Transformation of P. pastoris

All plasmids were linearized with *Sal*I to promote integration at the *his4* locus rather than the *AOX1* locus, thus enabling normal growth on methanol. Transformation of *P. pastoris his4* strain GS115 and selection of transformants was as known in the art.

### Fermentation of P. pastoris

Fed-batch fermentations were performed in 2.5-liter Bioflo 3000 fermenters (New Brunswick Scientific), essentially as described by Zhang et al. in Biotechnol Bioeng. 70 1-8 (2000). Minimal basal salts medium was used and no protease-inhibiting supplements were added. The pH was maintained at 3.0 throughout the fermentation by addition of ammonium hydroxide as base. The methanol fed-batch phase for protein production lasted three to four days. A homemade semiconductor gas sensor-controller was used to monitor the methanol level in the off-gas and to maintain a constant level of ∼0.2% (w/v) methanol in the broth. At the end of the fermentation, the cells were separated from the broth by centrifugation for 10 min. at 10,000 × g (RT) in an SLA-3000 rotor (Sorvall), and the supernatant was microfiltered.

### Protein purification

All centrifugation steps were performed in an SLA-1500 or SLA-3000 rotor (Sorvall) for 30 min. at 20,000 × g, and resuspension of protein pellets was always in Milli-Q water at 65 °C.
As a precaution, the cellfree broth was heated for 30 min. at 65 °C to melt possible gel structures formed by the recombinant protein. The pH was raised to 8.0 by addition of sodium hydroxide to allow precipitation of medium salts by centrifugation (RT). The protein was precipitated from the supernatant by addition of ammonium sulfate to 40% of saturation, followed by incubation on ice for 30 min. and centrifugation (4 °C). This precipitation procedure was repeated once, and 50 mM sodium chloride and 40% (v/v) acetone were added to the final resuspended pellet. After centrifugation (4 °C), the pellet was discarded and acetone was added to the supernatant up to 80% (v/v). The protein pellet obtained after centrifugation was air dried, resuspended, and desalted by extensive dialysis against Milli-Q water. The final product was lyophilized.

### SDS-PAGE and N-terminalprotein sequencing

The NuPAGE^{®} Novex^{®} system (Invitrogen) was used for SDS-PAGE, with 10% Bis-Tris gels, MES SDS running buffer and SeeBlue^{®} Plus2 pre-stained molecular mass markers. Gels were stained with Coomassie SimplyBlue SafeStain (Invitrogen). Blotting of proteins for N-terminal sequencing by Edman degradation was as described previously (Yeast 15, 1087-1096 (1999). Protein sequencing was performed by Midwest Analytical (St. Louis, MO).

### Mass spectrometry

Matrix-assisted laser desorption/ionization time-of-flight mass spectrometry (MALDI-TOF MS) was performed using an Ultraflex mass spectrometer (Bruker). Samples were prepared by the dried droplet method on a 600 µm AnchorChip target (Bruker), using 5 mg/ml 2,5-dihydroxyacetophenone, 1.5 mg/ml diammonium hydrogen citrate, 25% (v/v) ethanol and 1% (v/v) trifluoroacetic acid as matrix. Measurements (50 shots at 20 Hz) were made in the positive, linear mode, with the following parameters: ion source 1 20,000 V; ion source 2 18,450 V; lens 5,000 V, pulsed ion extraction 550 ns. Protein Calibration Standard II (Bruker) was used for external calibration.

### Circular dichroism spectrometry

Proteins were dissolved in 4.2 M sodium phosphate pH 3.0 at ∼0.2 or 100 mg/ml. Circular dichroic (CD) spectra were recorded using a J-715 spectropolarimeter (Jasco) set at the desired temperature. Pathlength was 1 mm for measurements at 0.2 mg/ml (260 to 190 nm) and 0.01 mm for measurements at 100 mg/ml (260 to 210 nm). Spectra were obtained as the average of ten consecutive scans using a scanning speed of 100 nm/min at a resolution of 0.2 nm. Melting curves from 20 to 65 °C were derived by increasing the temperature in steps of 5 or 10 °C, followed by equilibration at each temperature for 10 min. prior to scanning.

### Differential scanning calorimetry

0.5 ml degassed protein solutions (0.4 to 2.7 mM in 0.2 M sodium phosphate pH 3.0) were loaded into a MicroCal VP-DSC calorimeter at 45 °C. Protein solutions were equilibrated for 10h at 20 °C to allow complete helix formation. For melting curves, the temperature was increased from 5 to 65 °C at a scan rate of 15 °C/h.

### Rheometry

An Anton Paar Physica MCR301 rheometer, equipped with a stainless steel CC17 couette geometry, gap size: 0.71 mm, bob radius: 8.3 mm, and sample volume of 3 ml, was operated at an angular frequency of 1Hz and a strain of 0.1%. Protein solutions (2.2 mM in 0.2 M sodium phosphate pH 3.0) were heated to 45 °C, introduced in the geometry, and subsequently quenched to 20 °C to induce gel formation. Measurements were done at 20 and 5 °C. Melting of the gel was studied by increasing the temperature from 20 to 65 °C in steps of 2.5 °C that lasted 10 min. each.

### Controlled release

1 ml of TR₄T solution (0.2 g/ml) in PBS (10 mM phosphate, 150 mM NaCl, pH 7.4) with 10 mg/ml lysozyme was heated to 60 °C until homogeneity and then incubated at 20 °C overnight to allow gel formation. Subsequently, 1 ml of PBS was added to the gel and lysozym release was monitored at 20 and 37 °C for about two days. At various time points, 0.5 ml buffer was withdrawn for analysis and 0.5 ml fresh buffer was added. Lysozyme was quantified by size exclusion chromatography (Waters LC System, Superdex 75, elution in PBS, flow rate 1 ml/min) and comparison of the tryptophan fluorescence of the sample to a lysozyme standard.

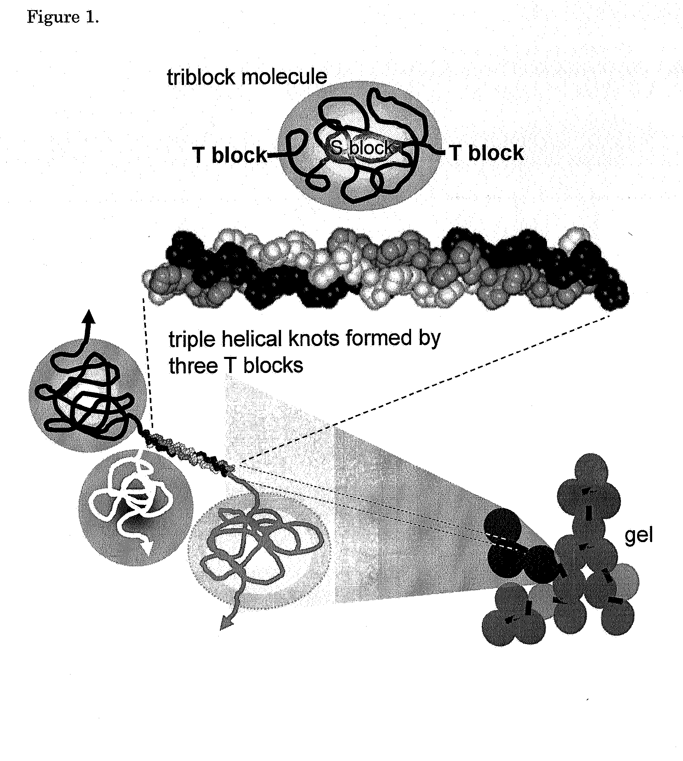

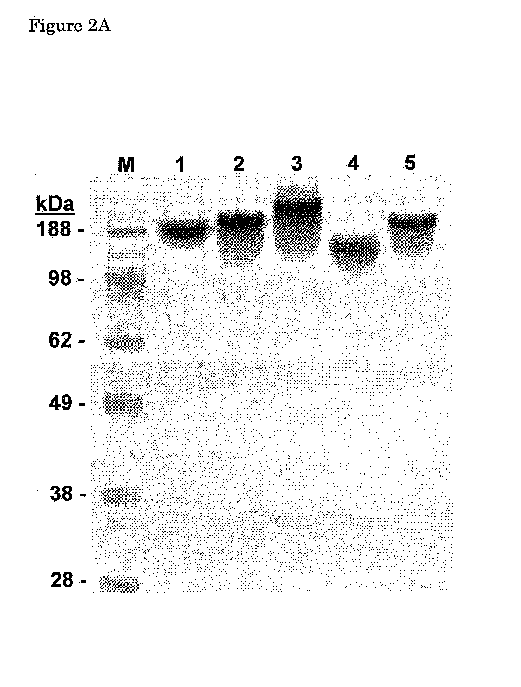

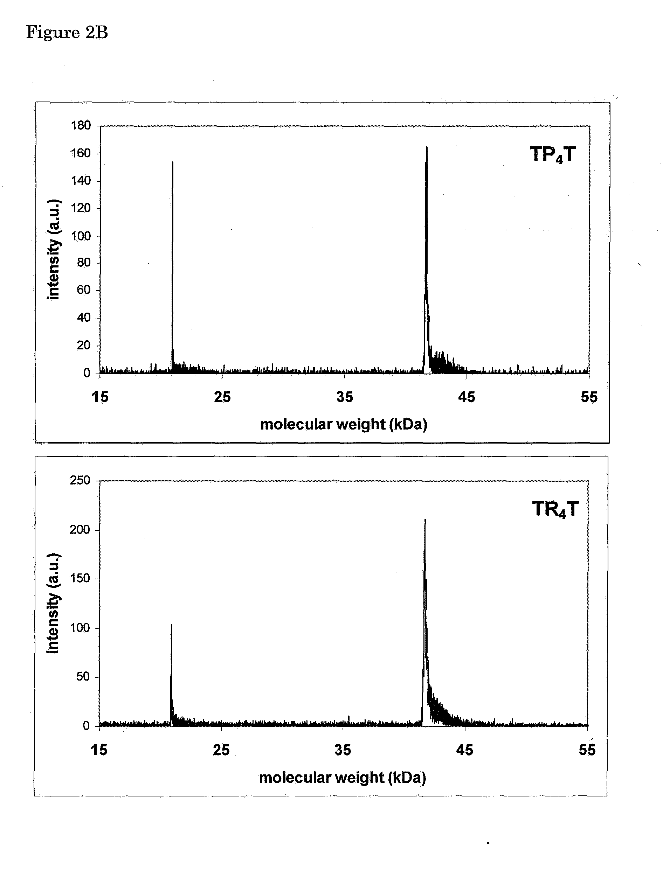

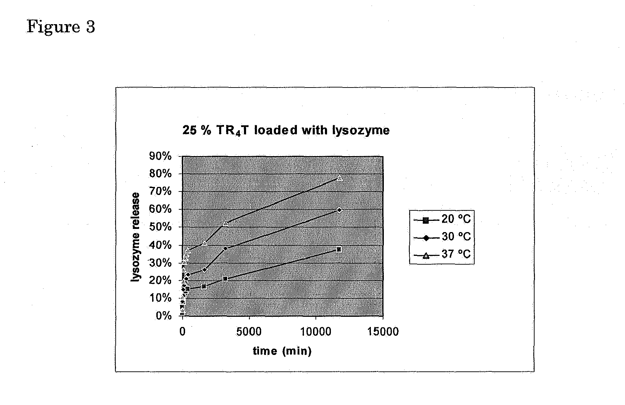

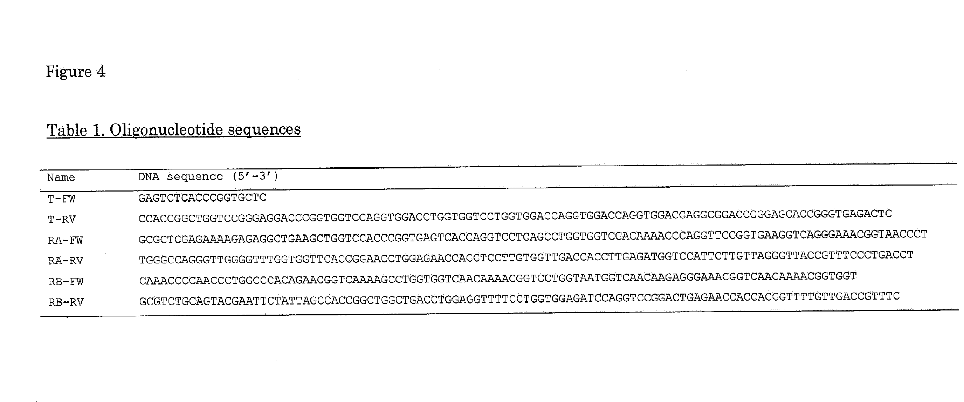

To show the suitability of the present gels for controlled release of for example protein drugs, the release of lysozyme was investigated in a pilot study (Fig. 3). After initial burst release of the loaded enzyme (10 mg/ml) during the first six hours, sustained release progressed during the monitoring period. As expected, the release at physiological temperature was higher than that at 20 °C. These data indicate that the present nanostructured gels are useful to be developed into suitable drug release systems.

The polypeptides produced have purity to the extent that the content of other proteins is approximately 0-3 wt.%(typically 1 %), the carbohydrate content is approximately 0-4 wt.% (typically 0.1-0.5 %), and the content of ammonium sulfate is approximately 0-15 wt.%.
The polypeptides have the following amino acid sequences.
(A) "TP₄T" Mw: 41 741 Da: 
GPPGAPGPPGPPGPPGPPGPPGPPGPPGPPGPAGEPGNPGSPGNQGQPGNKGSPGNPGQPGNEGQP
GQPGQNGQPGEPGSNGPQGSQGNPGKNGQPGSPGSQGSPGNQGSPGQPGNPGQPGEQGKPGNQGPA
GEPGNPGSPGNQGQPGNKGSPGNPGQPGNEGQPGQPGQNGQPGEPGSNGPQGSQGNPGKNGQPGSP
GSQGSPGNQGSPGQPGNPGQPGEQGKPGNQGPAGEPGNPGSPGNQGQPGNKGSPGNPGQPGNEGQP
GQPGQNGQPGEPGSNGPQGSQGNPGKNGQPGSPGSQGSPGNQGSPGQPGNPGQPGEQGKPGNQGPA
GEPGNPGSPGNQGQPGNKGSPGNPGQPGNEGQPGQPGQNGQPGEPGSNGPQGSQGNPGKNGQPGSP
GSGSPGNQGSPGQPGNPGQPGEQGKPGNQGPAGAPGPPGPPGPPGPPGPPGPPGPPGPPGPAGG
(B) "TR₄T" Mw: 41 741 Da: 
GPPGAPGPPGPPGPPGPPGPPGPPGPPGPPGPAGESPGPQPGGPQNPGSGEGQGNGNPNKNGPSQG
GQPQGGGSPGSGEPPNPNPGPQNGQKPGGQQNGPGNGQQEGNGQQNGGGSQSGPGSPPGKPPGQPA
GESPGPQPGGPQNPGSGEGQGNGNPNKNGPSQGGQPQGGGSPGSGEPPNPNPGPQNGQKPGGQQNG
PGNGQQEGNGQQNGGGSQSGPGSPPGKPPGQPAGESPGPQPGGPQNPGSGEGQGNGNPNKNGPSQG
GQPQGGGSPGSGEPPNPNPGPQNGQKPGGQQNGPGNGQQEGNGQQNGGGSQSGPGSPPGKPPGQPA
GESPGPQPGGPQNPGSGEGQGNGNPNKNGPSQGGQPQGGGSPGSGEPPNPNPGPQNGQKPGGQQNG
PGNGQQEGNGQQNGGGSQSGPGSPPGKPPGQPAGAPGPPGPPGPPGPPGPPGPPGPPGPAGG
(C) "TP₈T" Mw: 78 175 Da: 
GPPGAPGPPGPPGPPGPPGPPGPPGPPGPPGPAGEPGNPGSPGNQGQPGNKGSPGNPGQPGNEGQP
GQPGQNGQPGEPGSNGPQGSQGNPGKNGQPGSPGSQGSPGNQGSPGQPGNPGQPGEQGKPGNQGPA
GEPGNPGSPGNQGQPGNKGSPGNPGQPGNEGQPGQPGQNGQPGEPGSNGPQGSQGNPGKNGQPGSP
GSQGSPGNQGSPGQPGNPGQPGEQGKPGNQGPAGEPGNPGSPGNQGQPGNKGSPGNPGQPGNEGQP
GQPGQNGQPGEPGSNGPQGSQGNPGKNGQPGSPGSQGSPGNQGSPGQPGNPGQPGEQGKPGNQGPA
GEPGNPGSPGNQGQPGNKGSPGNPGQPGNEGQPGQPGQNGQPGEPGSNGPQGSQGNPGKNGQPGSP
GSQGSPGNQGSPGQPGNPGQPGEQGKPGNQGPAGEPGNPGSPGNQGQPGNKGSPGNPGQPGNEGQP
GQPGQNGQPGEPGSNGPQGSQGNPGKNGQPGSPGSQGSPGNQGSPGQPGNPGQPGEQGKPGNQGPA
GEPGNPGSPGNQGQPGNKGSPGNPGQPGNEGQPGQPGQNGQPGEPGSNGPQGSQGNPGKNGQPGSP
GSQGSPGNQGSPGQPGNPGQPGEQGKPGNQGPAGEPGNPGSPGNQGQPGNKGSPGNPGQPGNEGQP
GQPGQNGQPGEPGSNGPQGSQGNPGKNGQPGSPGSQGSPGNQGSPGQPGNPGQPGEQGKPGNQGPA
GEPGNPGSPGNQGQPGNKGSPGNPGQPGNEGQPGQPGQNGQPGEPGSNGPQGSQGNPGKNGQPGSP
GSQGSPGNQGSPGQPGNPGQPGEQGKPGNQGPAGAPGPPGPPGPPGPPGPPGPPGPPGPPGPAGG
(D) "TR₈T" Mw: 78 175 Da: 
GPPGAPGPPGPPGPPGPPGPPGPPGPPGPPGPAGESPGPQPGGPQNPGSGEGQGNGNPNKNGPSQG
GQPQGGGSPGSGEPPNPNPGPQNGQKPGGQQNGPGNGQQEGNGQQNGGGSQSGPGSPPGKPPGQPA
GESPGPQPGGPQNPGSGEGQGNGNPNKNGPSQGGQPQGGGSPGSGEPPNPNPGPQNGQKPGGQQNG
PGNGQQEGNGQQNGGGSQSGPGSPPGKPPGQPAGESPGPQPGGPQNPGSGEGQGNGNPNKNGPSQG
GQPQGGGSPGSGEPPNPNPGPQNGQKPGGQQNGPGNGQQEGNGQQNGGGSQSGPGSPPGKPPGQPA
GESPGPQPGGPQNPGSGEGQGNGNPNKNGPSQGGQPQGGGSPGSGEPPNPNPGPQNGQKPGGQQNG
PGNGQQEGNGQQNGGGSQSGPGSPPGKPPGQPAGESPGPQPGGPQNPGSGEGQGNGNPNKNGPSQG
GQPQGGGSPGSGEPPNPNPGPQNGQKPGGQQNGPGNGQQEGNGQQNGGGSQSGPGSPPGKPPGQPA
GESPGPQPGGPQNPGSGEGQGNGNPNKNGPSQGGQPQGGGSPGSGEPPNPNPGPQNGQKPGGQQNG
PGNGQQEGNGQQNGGGSQSGPGSPPGKPPGQPAGESPGPQPGGPQNPGSGEGQGNGNPNKNGPSQG
GQPQGGGSPGSGEPPNPNPGPQNGQKPGGQQNGPGNGQQEGNGQQNGGGSQSGPGSPPGKPPGQPA
GESPGPQPGGPQNPGSGEGQGNGNPNKNGPSQGGQPQGGGSPGSGEPPNPNPGPQNGQKPGGQQNG
PGNGQQEGNGQQNGGGSQSGPGSPPGKPPGQPAGAPGPPGPPGPPGPPGPPGPPGPPGPPGPAGG
(E) "TP₁₂T" Mw: 114 610Da: 
GPPGAPGPPGPPGPPGPPGPPGPPGPPGPPGPAGEPGNPGSPGNQGQPGNKGSPGNPGQPGNEGQP
GQPGQNGQPGEPGSNGPQGSQGNPGKNGQPGSPGSQGSPGNQGSPGQPGNPGQPGEQGKPGNQGPA
GEPGNPGSPGNQGQPGNKGSPGNPGQPGNEGQPGQPGQNGQPGEPGSNGPQGSQGNPGKNGQPGSP
GSQGSPGNQGSPGQPGNPGQPGEQGKPGNQGPAGEPGNPGSPGNQGQPGNKGSPGNPGQPGNEGQP
GQPGQNGQPGEPGSNGPQGSQGNPGKNGQPGSPGSQGSPGNQGSPGQPGNPGQPGEQGKPGNQGPA
GEPGNPGSPGNQGQPGNKGSPGNPGQPGNEGQPGQPGQNGQPGEPGSNGPQGSQGNPGKNGQPGSP
GSQGSPGNQGSPGQPGNPGQPGEQGKPGNQGPAGEPGNPGSPGNQGQPGNKGSPGNPGQPGNEGQP
GQPGQNGQPGEPGSNGPQGSQGNPGKNGQPGSPGSQGSPGNQGSPGQPGNPGQPGEQGKPGNQGPA
GEPGNPGSPGNQGQPGNKGSPGNPGQPGNEGQPGQPGQNGQPGEPGSNGPQGSQGNPGKNGQPGSP
GSQGSPGNQGSPGQPGNPGQPGEQGKPGNQGPAGEPGNPGSPGNQGQPGNKGSPGNPGQPGNEGQP
GQPGQNGQPGEPGSNGPQGSQGNPGKNGQPGSPGSQGSPGNQGSPGQPGNPGQPGEQGKPGNQGPA
GEPGNPGSPGNQGQPGNKGSPGNPGQPGNEGQPGQPGQNGQPGEPGSNGPQGSQGNPGKNGQPGSP
GSQGSPGNQGSPGQPGNPGQPGEQGKPGNQGPAGEPGNPGSPGNQGQPGNKGSPGNPGQPGNEGQP
GQPGQNGQPGEPGSNGPQGSQGNPGKNGQPGSPGSQGSPGNQGSPGQPGNPGQPGEQGKPGNQGPA
GEPGNPGSPGNQGQPGNKGSPGNPGQPGNEGQPGQPGQNGQPGEPGSNGPQGSQGNPGKNGQPGSP
GSQGSPGNQGSPGQPGNPGQPGEQGKPGNQGPAGEPGNPGSPGNQGQPGNKGSPGNPGQPGNEGQP
GQPGQNGQPGEPGSNGPQGSQGNPGKNGQPGSPGSQGSPGNQGSPGQPGNPGQPGEQGKPGNQGPA
GEPGNPGSPGNQGQPGNKGSPGNPGQPGNEGQPGQPGQNGQPGEPGSNGPQGSQGNPGKNGQPGSP
GSQGSPGNQGSPGQPGNPGQPGEQGKPGNQGPAGAPGPPGPPGPPGPPGPPGPPGPPGPPGPAGG
(F) "TR₁₂T" Mw: 114 610Da:

GPPGAPGPPGPPGPPGPPGPPGPPGPPGPPGPAGESPGPQPGGPQNPGSGEGQGNGNPNKNGPSQG
GQPQGGGSPGSGEPPNPNPGPQNGQKPGGQQNGPGNGQQEGNGQQNGGGSQSGPGSPPGKPPGQPA
GESPGPQPGGPQNPGSGEGQGNGNPNKNGPSQGGQPQGGGSPGSGEPPNPNPGPQNGQKPGGQQNG
PGNGQQEGNGQQNGGGSQSGPGSPPGKPPGQPAGESPGPQPGGPQNPGSGEGQGNGNPNKNGPSQG
GQPQGGGSPGSGEPPNPNPGPQNGQKPGGQQNGPGNGQQEGNGQQNGGGSQSGPGSPPGKPPGQPA
GESPGPQPGGPQNPGSGEGQGNGNPNKNGPSQGGQPQGGGSPGSGEPPNPNPGPQNGQKPGGQQNG
PGNGQQEGNGQQNGGGSQSGPGSPPGKPPGQPAGESPGPQPGGPQNPGSGEGQGNGNPNKNGPSQG
GQPQGGGSPGSGEPPNPNPGPQNGQKPGGQQNGPGNGQQEGNGQQNGGGSQSGPGSPPGKPPGQPA
GESPGPQPGGPQNPGSGEGQGNGNPNKNGPSQGGQPQGGGSPGSGEPPNPNPGPQNGQKPGGQQNG
PGNGQQEGNGQQNGGGSQSGPGSPPGKPPGQPAGESPGPQPGGPQNPGSGEGQGNGNPNKNGPSQG
GQPQGGGSPGSGEPPNPNPGPQNGQKPGGQQNGPGNGQQEGNGQQNGGGSQSGPGSPPGKPPGQPA
GESPGPQPGGPQNPGSGEGQGNGNPNKNGPSQGGQPQGGGSPGSGEPPNPNPGPQNGQKPGGQQNG
PGNGQQEGNGQQNGGGSQSGPGSPPGKPPGQPAGESPGPQPGGPQNPGSGEGQGNGNPNKNGPSQG
GQPQGGGSPGSGEPPNPNPGPQNGQKPGGQQNGPGNGQQEGNGQQNGGGSQSGPGSPPGKPPGQPA
GESPGPQPGGPQNPGSGEGQGNGNPNKNGPSQGGQPQGGGSPGSGEPPNPNPGPQNGQKPGGQQNG
PGNGQQEGNGQQNGGGSQSGPGSPPGKPPGQPAGESPGPQPGGPQNPGSGEGQGNGNPNKNGPSQG
GQPQGGGSPGSGEPPNPNPGPQNGQKPGGQQNGPGNGQQEGNGQQNGGGSQSGPGSPPGKPPGQPA
GESPGPQPGGPQNPGSGEGQGNGNPNKNGPSQGGQPQGGGSPGSGEPPNPNPGPQNGQKPGGQQNG
PGNGQQEGNGQQNGGGSQSGPGSPPGKPPGQPAGAPGPPGPPGPPGPPGPPGPPGPPGPPGPAGG

### Example 2

In analogous manner, polypeptides were prepared having Trimerizing Blocks G comprising (Pro-Gly-Pro)₁₆.
(A) "T₂P₄T₂": 
GPPGAPGPPGPPGPPGPPGPPGPPGPPGPPGPPGPPGPPGPPGPPGPPGPPGPAGEPGNPGSPGNQ
GQPGNKGSPGNPGQPGNEGQPGQPGQNGQPGEPGSNGPQGSQGNPGKNGQPGSPGSQGSPGNQGSP
GQPGNPGQPGEQGKPGNQGPAGEPGNPGSPGNQGQPGNKGSPGNPGQPGNEGQPGQPGQNGQPGEP
GSNGPQGSQGNPGKNGQPGSPGSQGSPGNQGSPGQPGNPGQPGEQGKPGNQGPAGEPGNPGSPGNQ
GQPGNKGSPGNPGQPGNEGQPGQPGQNGQPGEPGSNGPQGSQGNPGKNGQPGSPGSQGSPGNQGSP
GQPGNPGQPGEQGKPGNQGPAGEPGNPGSPGNQGQPGNKGSPGNPGQPGNEGQPGQPGQNGQPGEP
GSNGPQGSQGNPGKNGQPGSPGSQGSPGNQGSPGQPGNPGQPGEQGKPGNQGPAGAPGPPGPPGPP
GPPGPPGPPGPPGPPGPPGPPGPPGPPGPPGPPGPPGPAGG
(B) "T₂R₄T₂"- 
GPPGAPGPPGPPGPPGPPGPPGPPGPPGPPGPPGPPGPPGPPGPPGPPGPAGESPGPQPGGPQ
NPGSGEGQGNGNPNKNGPSQGGQPQGGGSPGSGEPPNPNPGPQNGQKPGGQQNGPGNGQQEGNGQQ
NGGGSQSGPGSPPGKPPGQPAGESPGPQPGGPQNPGSGEGQGNGNPNKNGPSQGGQPQGGGSPGSG
EPPNPNPGPQNGQKPGGQQNGPGNGQQEGNGQQNGGGSQSGPGSPPGKPPGQPAGESPGPQPGGPQ
NPGSGEGQGNGNPNKNGPSQGGQPQGGGSPGSGEPPNPNPGPQNGQKPGGQQNGPGNGQQEGNGQQ
NGGGSQSGPGSPPGKPPGQPAGESPGPQPGGPQNPGSGEGQGNGNPNKNGPSQGGQPQGGGSPGSG
EPPNPNPGPQNGQKPGGQQNGPGNGQQEGNGQQNGGGSQSGPGSPPGKPPGQPAGAPGPPGPPGPP
GPPGPPGPPGPPGPPGPPGPPGPPGPPGPPGPPGPAGG
(C) "T₂P₈T₂": 
GPPGAPGPPGPPGPPGPPGPPGPPGPPGPPGPPGPPGPPGPPGPPGPPGPAGEPGNPGSPGNQ
GQPGNKGSPGNPGQPGNEGQPGQPGQNGQPGEPGSNGPQGSQGNPGKNGQPGSPGSQGSPGNQGSP
GQPGNPGQPGEQGKPGNQGPAGEPGNPGSPGNQGQPGNKGSPGNPGQPGNEGQPGQPGQNGQPGEP
GSNGPQGSQGNPGKNGQPGSPGSQGSPGNQGSPGQPGNPGQPGEQGKPGNQGPAGEPGNPGSPGNQ
GQPGNKGSPGNPGQPGNEGQPGQPGQNGQPGEPGSNGPQGSQGNPGKNGQPGSPGSQGSPGNQGSP
GQPGNPGQPGEQGKPGNQGPAGEPGNPGSPGNQGQPGNKGSPGNPGQPGNEGQPGQPGQNGQPGEP
GSNGPQGSQGNPGKNGQPGSPGSQGSPGNQGSPGQPGNPGQPGEQGKPGNQGPAGEPGNPGSPGNQ
GQPGNKGSPGNPGQPGNEGQPGQPGQNGQPGEPGSNGPQGSQGNPGKNGQPGSPGSQGSPGNQGSP
GQPGNPGQPGEQGKPGNQGPAGEPGNPGSPGNQGQPGNKGSPGNPGQPGNEGQPGQPGQNGQPGEP
GSNGPQGSQGNPGKNGQPGSPGSQGSPGNQGSPGQPGNPGQPGEQGKPGNQGPAGEPGNPGSPGNQ
GQPGNKGSPGNPGQPGNEGQPGQPGQNGQPGEPGSNGPQGSQGNPGKNGQPGSPGSQGSPGNQGSP
GQPGNPGQPGEQGKPGNQGPAGEPGNPGSPGNQGQPGNKGSPGNPGQPGNEGQPGQPGQNGQPGEP
GSNGPQGSQGNPGKNGQPGSPGSQGSPGNQGSPGQPGNPGQPGEQGKPGNQGPAGAPGPPGPPGPP
GPPGPPGPPGPPGPPGPPGPPGPPGPPGPPGPPGPPGPAGG
(D) "T₂R₈T₂": 
GPPGAPGPPGPPGPPGPPGPPGPPGPPGPPGPPGPPGPPGPPGPPGPPGPPGPAGESPGPQPGGPQ
NPGSGEGQGNGNPNKNGPSQGGQPQGGGSPGSGEPPNPNPGPQNGQKPGGQQNGPGNGQQEGNGQQ
NGGGSQSGPGSPPGKPPGQPAGESPGPQPGGPQNPGSGEGQGNGNPNKNGPSQGGQPQGGGSPGSG
EPPNPNPGPQNGQKPGGQQNGPGNGQQEGNGQQNGGGSQSGPGSPPGKPPGQPAGESPGPQPGGPQ
NPGSGEGQGNGNPNKNGPSQGGQPQGGGSPGSGEPPNPNPGPQNGQKPGGQQNGPGNGQQEGNGQQ
NGGGSQSGPGSPPGKPPGQPAGESPGPQPGGPQNPGSGEGQGNGNPNKNGPSQGGQPQGGGSPGSG
EPPNPNPGPQNGQKPGGQQNGPGNGQQEGNGQQNGGGSQSGPGSPPGKPPGQPAGESPGPQPGGPQ
NPGSGEGQGNGNPNKNGPSQGGQPQGGGSPGSGEPPNPNPGPQNGQKPGGQQNGPGNGQQEGNGQQ
NGGGSQSGPGSPPGKPPGQPAGESPGPQPGGPQNPGSGEGQGNGNPNKNGPSQGGQPQGGGSPGSG
EPPNPNPGPQNGQKPGGQQNGPGNGQQEGNGQQNGGGSQSGPGSPPGKPPGQPAGESPGPQPGGPQ
NPGSGEGQGNGNPNKNGPSQGGQPQGGGSPGSGEPPNPNPGPQNGQKPGGQQNGPGNGQQEGNGQQ
NGGGSQSGPGSPPGKPPGQPAGESPGPQPGGPQNPGSGEGQGNGNPNKNGPSQGGQPQGGGSPGSG
EPPNPNPGPQNGQKPGGQQNGPGNGQQEGNGQQNGGGSQSGPGSPPGKPPGQPAGAPGPPGPPGPP
GPPGPPGPPGPPGPPGPPGPPGPPGPPGPPGPPGPPGPAGG
(E) "T₂P₁₂T₂": 
GPPGAPGPPGPPGPPGPPGPPGPPGPPGPPGPPGPPGPPGPPGPPGPPGPAGEPGNPGSPGNQ
GQPGNKGSPGNPGQPGNEGQPGQPGQNGQPGEPGSNGPQGSQGNPGKNGQPGSPGSQGSPGNQGSP
GQPGNPGQPGEQGKPGNQGPAGEPGNPGSPGNQGQPGNKGSPGNPGQPGNEGQPGQPGQNGQPGEP
GSNGPQGSQGNPGKNGQPGSPGSQGSPGNQGSPGQPGNPGQPGEQGKPGNQGPAGEPGNPGSPGNQ
GQPGNKGSPGNPGQPGNEGQPGQPGQNGQPGEPGSNGPQGSQGNPGKNGQPGSPGSQGSPGNQGSP
GQPGNPGQPGEQGKPGNQGPAGEPGNPGSPGNQGQPGNKGSPGNPGQPGNEGQPGQPGQNGQPGEP
GSNGPQGSQGNPGKNGQPGSPGSQGSPGNQGSPGQPGNPGQPGEQGKPGNQGPAGEPGNPGSPGNQ
GQPGNKGSPGNPGQPGNEGQPGQPGQNGQPGEPGSNGPQGSQGNPGKNGQPGSPGSQGSPGNQGSP
GQPGNPGQPGEQGKPGNQGPAGEPGNPGSPGNQGQPGNKGSPGNPGQPGNEGQPGQPGQNGQPGEP
GSNGPQGSQGNPGKNGQPGSPGSQGSPGNQGSPGQPGNPGQPGEQGKPGNQGPAGEPGNPGSPGNQ
GQPGNKGSPGNPGQPGNEGQPGQPGQNGQPGEPGSNGPQGSQGNPGKNGQPGSPGSQGSPGNQGSP
GQPGNPGQPGEQGKPGNQGPAGEPGNPGSPGNQGQPGNKGSPGNPGQPGNEGQPGQPGQNGQPGEP
GSNGPQGSQGNPGKNGQPGSPGSQGSPGNQGSPGQPGNPGQPGEQGKPGNQGPAGEPGNPGSPGNQ
GQPGNKGSPGNPGQPGNEGQPGQPGQNGQPGEPGSNGPQGSQGNPGKNGQPGSPGSQGSPGNQGSP
GQPGNPGQPGEQGKPGNQGPAGEPGNPGSPGNQGQPGNKGSPGNPGQPGNEGQPGQPGQNGQPGEP
GSNGPQGSQGNPGKNGQPGSPGSQGSPGNQGSPGQPGNPGQPGEQGKPGNQGPAGEPGNPGSPGNQ
GQPGNKGSPGNPGQPGNEGQPGQPGQNGQPGEPGSNGPQGSQGNPGKNGQPGSPGSQGSPGNQGSP
GQPGNPGQPGEQGKPGNQGPAGEPGNPGSPGNQGQPGNKGSPGNPGQPGNEGQPGQPGQNGQPGEP
GSNGPQGSQGNPGKNGQPGSPGSQGSPGNQGSPGQPGNPGQPGEQGKPGNQGPAGAPGPPGPPGPP
GPPGPPGPPGPPGPPGPPGPPGPPGPPGPPGPPGPPGPAGG
(F) "T₂R₁₂T₂":

GPPGAPGPPGPPGPPGPPGPPGPPGPPGPPGPPGPPGPPGPPGPPGPPGPPGPAGESPGPQPGGPQ
NPGSGEGQGNGNPNKNGPSQGGQPQGGGSPGSGEPPNPNPGPQNGQKPGGQQNGPGNGQQEGNGQQ
NGGGSQSGPGSPPGKPPGQPAGESPGPQPGGPQNPGSGEGQGNGNPNKNGPSQGGQPQGGGSPGSG
EPPNPNPGPQNGQKPGGQQNGPGNGQQEGNGQQNGGGSQSGPGSPPGKPPGQPAGESPGPQPGGPQ
NPGSGEGQGNGNPNKNGPSQGGQPQGGGSPGSGEPPNPNPGPQNGQKPGGQQNGPGNGQQEGNGQQ
NGGGSQSGPGSPPGKPPGQPAGESPGPQPGGPQNPGSGEGQGNGNPNKNGPSQGGQPQGGGSPGSG
EPPNPNPGPQNGQKPGGQQNGPGNGQQEGNGQQNGGGSQSGPGSPPGKPPGQPAGESPGPQPGGPQ
NPGSGEGQGNGNPNKNGPSQGGQPQGGGSPGSGEPPNPNPGPQNGQKPGGQQNGPGNGQQEGNGQQ
NGGGSQSGPGSPPGKPPGQPAGESPGPQPGGPQNPGSGEGQGNGNPNKNGPSQGGQPQGGGSPGSG
EPPNPNPGPQNGQKPGGQQNGPGNGQQEGNGQQNGGGSQSGPGSPPGKPPGQPAGESPGPQPGGPQ
NPGSGEGQGNGNPNKNGPSQGGQPQGGGSPGSGEPPNPNPGPQNGQKPGGQQNGPGNGQQEGNGQQ
NGGGSQSGPGSPPGKPPGQPAGESPGPQPGGPQNPGSGEGQGNGNPNKNGPSQGGQPQGGGSPGSG
EPPNPNPGPQNGQKPGGQQNGPGNGQQEGNGQQNGGGSQSGPGSPPGKPPGQPAGESPGPQPGGPQ
NPGSGEGQGNGNPNKNGPSQGGQPQGGGSPGSGEPPNPNPGPQNGQKPGGQQNGPGNGQQEGNGQQ
NGGGSQSGPGSPPGKPPGQPAGESPGPQPGGPQNPGSGEGQGNGNPNKNGPSQGGQPQGGGSPGSG
EPPNPNPGPQNGQKPGGQQNGPGNGQQEGNGQQNGGGSQSGPGSPPGKPPGQPAGESPGPQPGGPQ
NPGSGEGQGNGNPNKNGPSQGGQPQGGGSPGSGEPPNPNPGPQNGQKPGGQQNGPGNGQQEGNGQQ
NGGGSQSGPGSPPGKPPGQPAGESPGPQPGGPQNPGSGEGQGNGNPNKNGPSQGGQPQGGGSPGSG
EPPNPNPGPQNGQKPGGQQNGPGNGQQEGNGQQNGGGSQSGPGSPPGKPPGQPAGAPGPPGPPGPP
GPPGPPGPPGPPGPPGPPGPPGPPGPPGPPGPPGPPGPAGG

## Claims

**1.** A block co-polypeptide comprising at least two blocks T and at least one block S, with blocks T and S alternating, wherein each T independently denotes a Trimerizing Block being a polypeptide block capable of forming a thermoreversible collagen-like triple helix structure, and wherein each S independently denotes a Spacer Block being a polypeptide block not intrinsically capable of forming a triple helix structure.

**2.** A block co-polypeptide according to claim 1, wherein one or more of the Trimerizing Blocks, each independently, are built such that glycine consistently occurs in every third position throughout the Trimerizing Block, using amino acid triplets selected from the group consisting of (Gly- Xaa - Yaa), (Yaa -Gly- Xaa), and (Xaa - Yaa -Gly), wherein Xaa and Yaa are amino acids and wherein the sequence Xaa-Yaa is selected, independently for each amino acid triplet, from the group consisting of PR, PP, PM, PI, PQ, EP, PA, ER, PV, PE, QR, KR, PT, KP, KQ, RP, QP, DP, EM, AR, RR, the letter designations being in accordance with the conventional one-letter nomenclature for amino acids.

**3.** A block co-polypeptide according to claim 1, wherein one or more of the Trimerizing Blocks, each independently, are built such that glycine consistently occurs in every third position throughout the Trimerizing Block, using pairs of amino acid triplets selected from the group consisting of (Gly-Xaa¹-Yaa¹- Gly-Xaa²-Yaa²), (Yaa¹-Gly-Xaa¹ - Yaa²-Gly-Xaa²) and (Xaa¹-Yaa¹-Gly- Xaa2-Yaa2-Gly) wherein Xaa¹, Xaa², Yaa¹ and Yaa² are amino acids and wherein the sequence pair (Xaa¹-Yaa¹, Xaa²-Yaa²) is selected, independently for each amino acid triplet pair, from the group consisting of (PR,EP), (PK,EP), (PE,KP), and (PE,RP), (PR,DP), (PK,DP), and (PD,KP, (PD,RP), provided that in the case of mixtures the charge occurring in the N-terminal part of any double triplet is opposite to the charge occurring in the C-terminal part of the preceding double triplet.

**4.** A block co-polypeptide according to claim 1, wherein one or more of the Trimerizing Blocks, each independently, are built such that glycine consistently occurs in every third position throughout the Trimerizing Block, using amino acid triplets selected from the group consisting of (Gly- Xaa - Yaa), (Yaa -Gly- Xaa), and (Xaa - Yaa -Gly), wherein Xaa and Yaa are amino acids and wherein the sequence Xaa-Yaa is selected, independently for each amino acid triplet, from the group consisting of PP, AP, EP, KP, RP, QP, DP, LP, VP, MP, IP, NP, SP, and wherein the proline in the Yaa position can optionally but not necessarily be modified into 4-hydroxy-proline.

**5.** A block co-polypeptide according to any one of the preceding claims, wherein the Spacer Blocks each independently have a gelatin-like amino acid composition or a sequence assuming an unordered conformation.

**5.** A block co-polypeptide according to any one of the preceding claims, wherein the Trimerizing Blocks each independently have a length of from 6 to 90 amino acids, preferably of from 9 to 60 amino acids, and most preferably of from 18 to 48 amino acids.

**6.** A block co-polypeptide according to any one of the preceding claims, wherein the Spacer Blocks S each independently have a length of from 50 to 3000 amino acids, preferably of from 200 to 1200 amino acids.

**7.** A block co-polypeptide according to any one of the preceding claims, wherein the Spacer Blocks S each independently comprise at least 5 % of proline, preferably at least 10% of proline, more preferably at least 20%, the percentage calculated on the basis of the total number of amino acid residues in the Spacer Block S including glycine.

**8.** A block co-polypeptide according to any one of the preceding claims, wherein the Spacer Blocks S each independently comprise from 10 to 25% of proline calculated on the basis of the number of amino acid residues in the Spacer Block S including glycine.

**9.** A block co-polypeptide according to any one of the preceding claims, wherein the formation of collagen-like triple helical structures is suppressed in one or more of the Spacer Blocks S, each independently, by avoidance of the occurrence of glycine in the majority of those positions that are separated from another glycine by two intermediate residues in the amino acid sequence of the Spacer Blocks S concerned, irrespective of the amino acid composition of the Spacer Blocks S concerned.

**10.** A block co-polypeptide according to any one of the preceding claims, wherein 50-100% of the amino acid residues Xaa and Yaa in the Trimerizing Blocks is proline.

**11.** A block co-polypeptide according to any one of the preceding claims, comprising more than one set of Trimerizing Blocks T, each set having a different melting temperature and all T blocks that are member of the same set having the same melting temperature, so as to provide gels with sets of blocks T melting at different temperatures, thereby allowing partial melting of the gel and modifying the effective behaviour and crosslink density of the gel without destroying the gel as a whole.

**12.** A block co-polypeptide according to any one of the preceding claims, wherein one or more of the Spacer Blocks comprise, each independently, one or more of a desired functionality selected from the group consisting of (a) peptide domains that bind to animal cell integrins, (b) peptide domains that bind to other animal cell surface structures (c) growth factor or growth factor-like domains, (d) antimicrobial sequences, (e) domains that interact with active agents, (f) polypeptide domains that serve as ligands for biological targets such as - but not limited to - single chain antibodies, (g) domains with a biological signalling function, (h) other bioactive domains and (i) combinations thereof.

**13.** A hydrogel comprising a block co-polypeptide according to any one of the preceding claims.

**14.** A method of making a hydrogel according to claim 13, wherein a block co-polypeptide in accordance with any one of the claims 1-12 is brought in aqueous suspension at a temperature above the gelation temperature, followed by cooling to below the gelation temperature.

**15.** A use of a hydrogel according to claim 13, or obtainable by a method according to claim 14, as a substitute for natural gelatin.

**16.** A use of a hydrogel according to claim 13, or obtainable by a method according to claim 14, in a product selected from the group consisting of , encapsulants, stabilizing agents, film-forming agents, moisturizing agents, emulsifiers, thickening agents, gelling agents, colloidal agents, adhesive agents, flocculating agents, and refining agents.

**17.** A pharmaceutical composition comprising a hydrogel according to claim 13, or obtainable by a method according to claim 14.

**18.** A pharmaceutical composition according to claim 17, wherein the hydrogel comprises a block co-polypeptide according to any one of the claims 1-12, for use in a method for the controlled release of a drug, the drug being released as a result of melting of Trimerizing Blocks T with lower melting temperature than other Trimerizing Blocks T.

**19.** An edible composition comprising a hydrogel according to claim 13, or obtainable by a method according to claim 14.

**20.** The use of a block co-polypeptide according to claim 1 through 12 as a carrier for the controlled release of a drug.

**21.** The use of a block co-polypeptide according to any one of the claims 1-11 in an application selected from the group consisting of cosmetics, photography and photoprinting, photographic prints of digital pictures, restorable surgery materials, coating of implants such as sensors, pumps, stents, artificial veins, artificial heart valves; injectable tissue reinforcing materials, scaffolds for grafting or seeding cells or tissue materials for tissue engineering and tissue regeneration; wound dressing materials, burn dressing materials, and plasma expanders.

**22.** A method for making a block copolypeptide according to of any of the claims 1-12, using recombinant technology involving the expression of DNA encoding for the block-copolypeptide in a host cell, wherein the block copolypeptide is secreted to the extracellular medium, even though the block copolypeptide is capable of forming collagen-like triple helices and hydrogels.

**23.** A method of making a block co-polypeptide according to any one of the claims 1-12, using recombinant technology involving the expression of DNA encoding for the block-copolypeptide in a host cell, wherein the length of the Trimerizing Blocks T, at given amino acid compositions and sequences of the Trimerizing Blocks T, is tuned, each independently, so as to tune the melting temperature of said Trimerizing Blocks.
